# EUROPÄISCHE PATENTSCHRIFT

(11) **EP 1 627 646 B1**
(45) Veröffentlichungstag und Bekanntmachung des Hinweises auf die Patenterteilung: **03.12.2008**
(21) Anmeldenummer: 05107508.3
(22) Anmeldetag: 16.08.2005
(51) Int. Cl.: A61L 2/16, A61L 2/18, C11D 1/825, A61K 31/14

(54) **Reinigungs- und Desinfektionsmittel für medizinische Instrumente mit verbesserter Wirksamkeit gegen Hepatitis-B-Viren**
Cleaning and disinfecting agent for medical instruments with improved effectiveness against Hepatitis B Virus
Composition de nettoyage et désinfection pour instruments médicaux possédant une efficacité améliorée contre le virus de l'hépatite B

(30) Priorität: 16.08.2004 DE 102004039727
(43) Veröffentlichungstag der Anmeldung: 22.02.2006
(73) Patentinhaber: Bode Chemie GmbH & Co. KG, 22525 Hamburg (DE)
(72) Erfinder: Bloß, Richard, Dr., 25462, Rellingen (DE); Fehling, Thomas, 22305, Hamburg (DE); Meyer, Stephanie, 20535 Hamburg (DE)
(74) Vertreter: Rabanus, Birgit

(56) Entgegenhaltungen:
- EP-A- 0 848 907
- EP-A- 1 126 012
- EP-A- 1 138 203
- WO-A-95/31962
- WO-A-03/099006

## Beschreibung

Gegenstand der Erfindung ist eine mikrobizide Zubereitung mit einem Gehalt an Isotridecanolethoxylat - und Dimethyldecyloxethylammoniumpropionat sowie deren Verwendung als Reinigungs- und Desinfektionsmittel für medizinische Instrumente. Die erfindungsgemäße Zubereitung besitzt eine besondere Hepatitis-B-(HBV)-Wirksamkeit neben einer ausgeglichenen bakteriziden und fungiziden Wirksamkeit. Die Zubereitung liegt entweder als Konzentrat vor, welches vor der Anwendung mit Wasser verdünnt wird, oder als fertige Gebrauchslösung.

Es ist bekannt, daß quaternäre Ammoniumverbindungen eine hohe antimikrobielle Aktivität und eine relativ geringe Toxizität aufweisen. Deswegen, und auch wegen ihres geringen Geruchs werden sie seit vielen Jahren in einer breiten Produktpalette zur Desinfektion in den Bereichen des Gesundheitswesens, der Industrie, etc. eingesetzt. Das Wirkungsspektrum quaternärer Ammoniumverbindungen ist breit, weist jedoch auch Lücken auf, wie zum Beispiel bei Mycobakterien und unbehüllten Viren.

Deswegen werden oft Kombinationen von quaternären Ammoniumverbindungen mit anderen Wirkstoffen, wie z. B. Alkylaminen oder Aldehyden, eingesetzt.

Die Anwendungskonzentrationen und -zeiten (d. h. die Anwendungsbedingungen) von Desinfektionsmitteln werden üblicherweise so gewählt, dass alle relevanten Keime unter diesen Bedingungen um einen jeweils festgelegten Wert reduziert werden. Das bedeutet, dass der hartnäckigste (sog. "kritische") Keim die jeweiligen Anwendungsbedingungen diktiert. Das bedeutet auch, dass für eine Reihe weniger "kritischer" Keime die Anwendungsbedingungen zu hoch liegen können. Es ist daher wünschenswert, wenn die Anwendungsbedingungen für alle relevanten Keime möglichst dicht beieinander liegen.

Die Keimreduktion soll selbstverständlich nicht nur unter reinen (Labor-) Bedingungen ("clean conditions"), sondern auch unter praktischen Bedingungen ("dirty conditions") erfolgen. Im Bereich der Instrumentendesinfektion sind die typischen Verunreinigungen Blut, Fett und Eiweiß. Quaternäre Ammoniumverbindungen sind oberflächenaktiv und positiv geladen. Dementsprechend können sie sowohl auf negativ geladene Mikroorganismen als auch auf negativ geladene Verunreinigungen aufziehen. Dieses Aufziehen veringert entsprechend die mikrobizide Wirksamkeit der quaternären Ammoniumverbindungen. Es ist daher von Vorteil, wenn dieser Effekt möglichst gering ist oder anders ausgedrückt: wenn sich die Anwendungsbedingungen unter clean und dirty conditions nicht oder kaum unterscheiden.

Instrumentendesinfektionsmittel enthalten neben den mikrobiziden Wirkstoffen in den meisten Fällen auch Tenside, um eine ausreichende Reinigung zu erzielen. Zusammen mit quaternären Ammoniumverbindungen werden gern nichtionische Tenside verwendet.

Bestimmte nichtionische Tenside sind bekannt für ihre gute Reinigungswirkung bei geringer bis moderater Schaumneigung. Dieses geht beispielsweise aus den folgenden Patentschriften hervor:

Die DE-100 07 323-A1 beschreibt flüssige Reinigungsmittel für medizinische Instrumente, die mindestens ein nichtionisches Tensid und mindestens eine Aminosäure oder ein Aminosäurederivat (vorzugsweise Pyrrolidoncarbonsäure) enthalten. Als nichtionisches Tensid wird vorzugsweise Isotridecanolethoxylat genannt.

In der DE-100 28 998-A1 werden reinigende und desinfizierende Systeme für medizinische Instrumente beansprucht, welche neben den in der DE-100 07 323-A1 genannten Komponenten zussätzlich einen weiteren mikrobiziden Wirkstoff enthalten. Bevorzugtes Tensid ist auch hier Isotridecanolethoxylat.

In der DE 100 30 946 wird ein ähnliches System wie vorstehend beansprucht, wobei auch hier bevorzugtes Tensid Isotridecanolethoxylat ist.

Die EP-A-11260142 offenbart eine Zubereitung zum Reinigen und Desinfizieren von unbelebten Oberflächen, enthaltend als nichtionisches Tensid Isotridecanolethoxylat, wobei auf weitere nichtionische Tenside verzichtet wird, und quaternäre Ammonium-Verbindungen.

Für quaternäre Ammoniumverbindungen ist das Hepatitis-B (HBV)-Virus der kritische Keim. Unter dirty conditions benötigt man deutlich härtere Bedingungen zur Erzielung einer ausreichenden Reduktion als für andere Keime. Diese Bedingungen bestimmen dementsprechend die Anwendungsempfehlung für das Präparat über das ganze Keimspektrum.

Ein weiterer Nachteil bisheriger Instrumentendesinfektionsmittel auf der Basis quaternärer Ammoniumverbindungen mit guter Viruzidie ist der hohe pH-Wert der Anwendungslösungen, welcher eine Korrosionen der medizinischen Instrumente fördert.

Es war somit eine Aufgabe der vorliegenden Erfindung, eine Zubereitung auf der Basis quaternärer Ammoniumverbindungen zu finden, welche unter ausgeglichenen Anwendungsbedingungen eingesetzt werden kann und insbesondere für Hepatitis-B-Viren keine härteren Bedingungen benötigt als für andere Keime. Gleichzeitig sollte das Mittel aber auch wirksam gegen Bakterien und Pilze sein, und zwar geprüft nach EN-Normen gegenüber *Staphylococcus aureus, Eschericia coli, Pseudomonas aeruginosa, Proteus mirabilis und Candida albicans*. Zudem sollte der pH-Wert in der Anwendungslösung abweichend von üblichen Instrumentedesinfektionsmitteln neutral eingestellt sein, um die Neigung der Proteinkoagulation des Mittels zu minimieren, eine effektive Reinigung zu ermöglichen und die Verträglichkeit des Mittels gegenüber den üblichen in der Medizin eingesetzten Materialien zu gewährleisten.

Dementsprechend werden die erfindungsgemäßen Aufgaben überraschend gelöst durch eine Zubereitung nach Auspruch 1.

Die erfindungsgemäße mikrobizide Zubereitung besitzt eine sehr gute mikrobizide Wirkung gegen Hepatitis-B-Viren, ohne daß die Wirksamkeit gegenüber Bakterien und Pilzen verringert ist. Die Wirkung gegen Hepatitis-B-Viren tritt überraschender Weise nur dann ein, wenn die Zubereitung im Sinne der vorliegenden Erfindung keine weiteren nichtionischen Tenside enthält.

Die erfindungsgemäßen mikrobiziden Mittel können in Form von Konzentraten oder (verdünnten) Anwendungslösungen zur Desinfektion von unbelebten Oberflächen aller Art verwendet werden, insbesondere zur Desinfektion von chirurgischem Instrumentarium und Anasthesiematerialien, wie starren oder flexiblen Endoskopen etc.. Die Desinfektion kann nach allen bekannten üblichen Verfahren durch Behandeln der Oberfläche mit der Zusammensetzung erfolgen. Gegebenenfalls können die Gegenstände im Ultraschallbad mit der Zusammensetzung behandelt werden.

Da die Wirkungssteigerung insbesondere bei hoher Eiweißbelastung eintritt, ist es vorteilhaft im Sinne der vorliegenden Erfindung, wenn die erfindungsgemäße Zusammensetzung als Desinfektionsreiniger eingesetzt wird.

Liegt die erfindungsgemäße Zusammensetzung als Konzentrat vor, so kommt sie üblicherweise in Form wässriger oder wässrig-alkoholischer Lösungen zur Anwendung (Anwendungslösung). Dementsprechend betrifft die vorliegende Erfindung sowohl mikrobizide Mittel, welche in Form von Konzentraten vorliegen, als auch solche, welche bereits in Form von Anwendungslösungen vorliegen.

Die quaternäre Ammoniumverbindung im Sinne der voriegenden Erfindung ist Dimethyldecyloxethylammoniumpropionat.

Es ist vorteilhaft im Sinne der vorliegenden Erfindung, wenn die Konzentration der quaternären Ammoniumverbindung von 10 bis 30 Gew.-%, vorzugsweise von 15 bis 20 Gew.-%, jeweils bezogen auf das Gesamtgewicht der Zubereitung, gewählt wird.

Es ist vorteilhaft im Sinne der vorliegenden Erfindung, das verzweigkettige nichtionische Tensid in einer Gesamtkonzentration von 5 bis 20 Gew.-%, vorzugsweise von 5 bis 15 Gew.-%, jeweils bezogen auf die Gesamtmenge eines Konzentrats, einzusetzen.

Das erfindungsgemäße nichtionische Tensid ist C₁₃-Iso-Alkylethoxylat (7 EO), welches als einziges verzweigkettiges nichtionisches Tensid im Sinne der vorliegenden Erfindung eingesetzt wird.

Das erfindungsgemäße mikrobizide Mittel kann ferner bis zu 30 Gew.%, vorzugsweise bis zu 15 Gew.%, bezogen auf die Gesamtmenge des Konzentrats, eines geradkettigen oder verzweigten gesättigten Alkohols mit bis zu 8 Kohlenstoffatomen enthalten. Bevorzugte Alkohole sind Ethanol, 1 -Propanol und 2- Propanol.

Schließlich können dem mikrobiziden Mittel im Sinne der vorliegenden Erfindung noch übliche Parfüme, Farbstoffe und Korrosionsinhibitoren in einem Anteil von bis zu 5 Gew.%, vorzugsweise bis zu 2 Gew.% zugesetzt werden.

Das erfindungsgemäße mikrobizide Mittel kann als solches oder in Lösung in einem geeigneten Lösungsmittel oder Lösungsmittelgemisch zur Anwendung kommen. Dementsprechend betrifft die vorliegende Erfindung auch eine Anwendungslösung umfassend das oben beschriebene mikrobizide Mittel (Konzentrat) gelöst in einem geeigneten Lösungsmittel oder Lösungsmittelgemisch.

Die erfindungsgemäße Anwendungslösung enthält das oben beschriebene Konzentrat in einem Anteil von 0,1 bis 10 Gew. -%, vorzugsweise von 0,3 bis 5 Gew.-%, bezogen auf das Gesamtvolumen der Anwendungslösung (Anwendungskonzentration).

Zur Herstellung der Anwendungslösung wird das Konzentrat mit Wasser verdünnt.

Der pH-Wert des Konzentrates beträgt 5 bis 9 und wird durch pH-Regulatoren wie z. B. Zitronensäure, Milchsäure, Essigsäure sowie Natronlauge oder Kalilauge eingestellt. Die Anwendungslösung hat vorteilhaft einen pH-Wert von 5 bis 9, vorzugsweise von 6 bis 8.

Die vorliegende Erfindung wird durch die nachfolgenden Beispiele näher beschrieben, ohne jedoch darauf beschränkt zu sein.

### Beispiele:

Es sind nachfolgend sieben Rezepturen dargestellt. Rezeptur 4 und 5 (Vergleichsbeispiele) entsprechen typischen derzeit im Markt befindlichen Produkten des Standes der Technik, sowohl mit als auch ohne quaternäre Ammoniumverbindungen. Rezepturen 1, 2, 3 und 6 sind ebenfalls Vergleichsbeispiele, Rezeptur 7 ist eine erfindungsgemäße Rezeptur.

**Tabelle 1:**

| **Rezepturbestandteil** | **1** | **2** | **3** | **4V** | **5V** | **6** | **7** |
|---|---|---|---|---|---|---|---|
| Didecylmethyloxethyl ammoniumpropionat | 15,4 | 17,5 | 17,5 | 17,5 | 17,5 | 17,5 | 17,5 |
| C_{12- 18}-Alkylalkoxylat (EO/PO) | 6 | - | - | - | - | - | - |
| Polyoxyethylen-5-alkylamin | 2 | - | - | - | - | - | - |
| C₁₂₋₁₄-Alkylethoxylat (12 EO) | - | 8 | 6 | 4 | 5 | 2 | 0 |
| C₁₃ Isotridecanolethoxylat 7 EO | - | 0 | 2 | 4 | 5 | 6 | 8 |
| Korrosionsinhibitor | 0,35 | 0,35 | 0,35 | 0,35 | 0,35 | 0,35 | 0,35 |
| Lösemittel | 11,2 | 12 | 5 | 5 | 5 | 5 | 5 |
| Komplexbildner | 2 | 2 | 2 | 2 | 2 | 2 | 2 |
| Schaumregulatoren | 0,7 | 0,7 | 0,7 | 0,7 | 0,7 | 0,7 | 0,7 |
| pH-Regulator | 0,54 | 0,45 | 0,45 | 0,45 | 0,45 | 0,45 | 0,45 |
| Farbstoff | 0,005 | 0,005 | 0,005 | 0,005 | 0,005 | 0,005 | 0,005 |
| Parfüm | 0,4 | 0,4 | 0,4 | 0,4 | 0,4 | 0,4 | 0,4 |
| Wasser | 61,405 | 58,595 | 58,595 | 58,595 | 56,595 | 58,595 | 58,595 |

Der pH-Wert der Rezepturen wurde jeweils in gleicher Weise auf pH 7 eingestellt. Die Rezepturen wurden zunächst auf die üblichen Anwendungskonzentrationen (AWK) verdünnt und diese verdünnten Lösungen dann auf ihre Wirksamkeit gegen Hepatitis B Viren getestet. Die Ergebnisse sind nachfolgender Tabelle 2 zu entnehmen.

**Tabelle 2:**

| **HBV-Wirksamkeit** | **1** | **2** | **3** | **4V** | **5V** | **6** | **7** |
|---|---|---|---|---|---|---|---|
| 15 Minuten Einwirkzeit | 1,0% | 1,2% | 1,2% | 1,2% | 1,2% | 0,9% | 0,9% |
| 30 Minuten Einwirkzeit | 0,9% | 1,2% | 1,2% | 1,2% | 1,2% | 0,9% | 0,6% |
| 60 Minuten Einwirkzeit | 0,9% | 1,2% | 1,2% | 0,9% | 0,9% | 0,9% | 0,6% |

Die Untersuchungen wurden mit einer organischen Belastung unter dirty conditions durchgeführt.

Die Auswertung zeigt, dass die Rezepturen 2 und 3 eine geringe Wirksamkeit gegen Hepytistis B Viren haben. Die Rezepturn 1, 4 und 5 zeigen eine durchschnittliche, etwas verbesserte Wirksamkeit. Bei der Rezeptur 1 ist zusätzlich zu einem nichtionischen Tensid das HBV-wirksame Tensid Alkylaminethoxylat enthalten.
Bemerkenswert ist, das die Rezeptur 6 und im besonderen die Rezeptur 7 eine deutlich verbesserte Wirksamkeit gegen Hepatiris B Viren unter organischer Belastung aufweist.

Aber auch bei Überprüfung der HBV-Wirksamkeit ohne Belastung und unter clean conditions zeigt die Rezeptur 7 das beste Ergebnis, wie in der nachfolgenden Tabelle gezeigt werden kann.

**Tabelle 3:**

| **HBV-Wirksamkeit** | **1** | **2** | **3** | **4V** | **5V** | **6** | **7** |
|---|---|---|---|---|---|---|---|
| 15 Minuten Einwirkzeit | 1,0% | 0,9% | > 1,2% | > 1,2% | 1,2% | 1,2% | 0,6% |
| 30 Minuten Einwirkzeit | 0,9% | 0,9% | > 1,2% | 1,2% | 1,2% | 1,2% | 0,6% |
| 60 Minuten Einwirkzeit | 0,9% | 0,9% | > 1,2% | 1,2% | 1,2% | 1,2% | 0,6% |

Die Auswertung zeigt, dass die Rezepturen 3 und 4 die geringste Wirksamkeit gegen Hepatitis B Viren haben. Die Rezepturen 1, 2, 5 und 6 besitzen eine durchschnittliche bis etwas bessere Wirksamkeit. Bei der Rezeptur 1 ist zusätzlich zu einem nichtionischen Tensid das HBV-wirksame Tensid Alkylaminethoxylat enthalten.
Bemerkenswert ist, dass auch ohne organische oder mit geringer organischer Belastung (clean conditions) Belastung die Rezeptur 7 eine deutlich verbesserte Wirksamkeit gegen Hepatiris B Viren aufweist.

In den nachfolgenden Diagrammen sind nochmal die Ergebnisse unter starker organischer Belastung (dirty conditions) graphisch dargestellt.

Um eine Wirksamkeit bei Hebatitis B Viren zu erzielen, muss der festgestellte Wert der Counts unterhalb eines mittleren Wertes von 300 liegen.

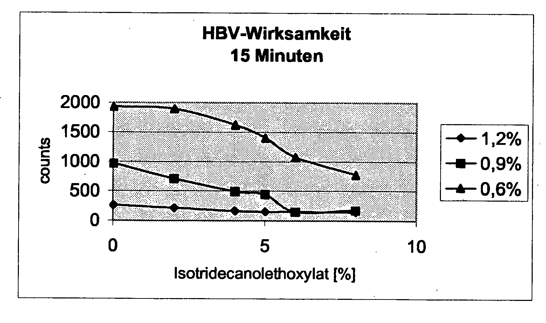

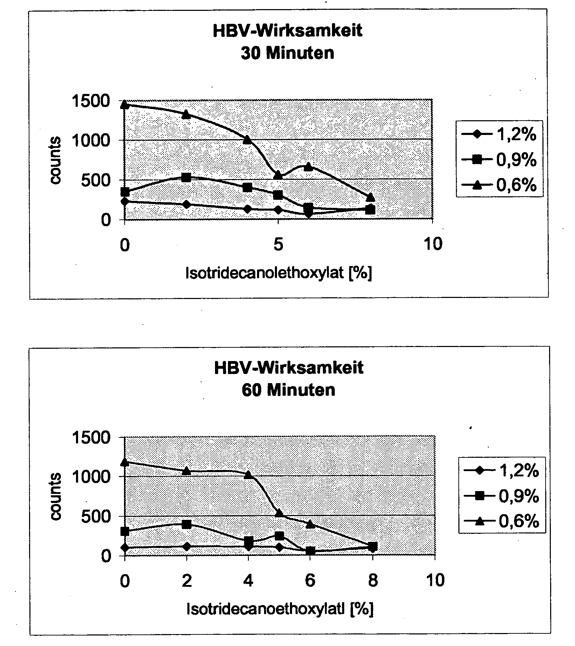

## Patentansprüche

1. Zubereitung zum Reinigen und Desinfizieren von unbelebten Oberflächen enthaltend Isotridecanolethoxylat,
wobei auf weitere nichtionische Tenside verzichtet wird, **gekennzeichnet dadurch, dass** sie Dimethyldecyloxethylammoniumpropionat enthält.

2. Zubereitung nach einem der vorhergehenden Ansprüche, **dadurch gekennzeichnet, dass** es einen pH-Wert zwischen 4 und 10 hat.

3. Zubereitung nach einem der vorhergehenden Ansprüche, **dadurch gekennzeichnet, dass** es in Form eines Konzentrats vorliegt, welches vor der Verwendung in Wasser zu einer Gebrauchslösung verdünn bar ist.

4. Zubereitung nach Anspruch 3, **dadurch gekennzeichnet, dass** die Konzentration des Dimethyldecyloxethylammoniumpropionat im Konzentrat von 10 bis 30 Gew.-%, vorzugsweise von 15 bis 20 Gew.-%, jeweils bezogen auf das Gesamtgewicht der Zubereitung beträgt.

5. Zubereitung nach Anspruch 3 oder 4, **dadurch gekennzeichnet, dass** die Konzentration von Isotridecanolethoxylat im Konzentrat von 5 bis 20 Gew.-%, vorzugsweise von 5 bis 15 Gew.-%, jeweils bezogen auf das Gesamtgewicht der Zubereitung, beträgt.

6. Zubereitung nach einem der vorhergehenden Ansprüche, **dadurch gekennzeichnet, dass** es zusätzlich bis zu 5 Gew.-% Korrosionsinhibitoren, Farbstoffe und/oder Parfümkomponenten enthält.

7. Verwendung einer Zubereitung gemäß einem der vorhergehenden Ansprüche zum Reinigen und Desinfizieren von medizinischen Instrumenten unter "clean" und/oder "dirty conditions".

## Claims

1. Preparation for the cleaning and disinfecting of inanimate surfaces comprising isotridecanol ethoxylate, where further nonionic surfactants are dispensed with, **characterized in that** it comprises dimethyldecyloxyethylammonium propionate.

2. Preparation according to one of the preceding claims, **characterized in that** it has a pH between 4 and 10.

3. Preparation according to one of the preceding claims, **characterized in that** it is in the form of a concentrate which, prior to use, can be diluted in water to give a use solution.

4. Preparation according to Claim 3, **characterized in that** the concentration of the dimethyldecyloxy-ethylammonium propionate in the concentrate is from 10 to 30 by weight, preferably from 15 to 20 by weight, in each case based on the total weight of the preparation.

5. Preparation according to Claim 3 or 4, **characterized in that** the concentration of isotridecanol ethoxylate in the concentrate is from 5 to 20 by weight, preferably from 5 to 15 by weight, in each case based on the total weight of the preparation.

6. Preparation according to one of the preceding claims, **characterized in that** it additionally comprises up to 5 by weight of corrosion inhibitors, dyes and/or perfume components.

7. Use of a preparation according to one of the preceding claims for the cleaning and disinfecting of medical instruments under "clean" and/or "dirty conditions".

## Revendications

1. Préparation destinée au nettoyage et à la désinfection de surfaces inanimées, contenant un produit d'éthoxylation d'isotridécanol, sans comprendre d'autres tensioactifs non ioniques, **caractérisée en ce qu'**elle contient du propionate de diméthyldécyloxyéthylammonium.

2. Préparation selon la revendication 1, **caractérisée en ce qu'**elle a un pH compris entre 4 et 10.

3. Préparation selon l'une quelconque des revendications précédentes, **caractérisée en ce qu'**elle se présente sous forme d'un concentré qui est avant l'emploi diluable dans de l'eau pour donner une solution prête à l'emploi.

4. Préparation selon la revendication 3, **caractérisée en ce que** la concentration du propionate de diméthyldécyloxyéthylammonium dans le concentré va de 10 à 30 % en poids, de préférence de 15 à 20 % en poids, chaque fois par rapport au poids total de la préparation.

5. Préparation selon la revendication 3 ou 4, **caractérisée en ce que** la concentration du produit d'éthoxylation d'isotridécanol dans le concentré va de 5 à 20 % en poids, de préférence de 5 à 15 % en poids, chaque fois par rapport au poids total de la préparation.

6. Préparation selon l'une quelconque des revendications précédentes, **caractérisée en ce qu'**elle contient en outre jusqu'à 5 % en poids d'inhibiteurs de corrosion, de colorants et/ou de composants de type parfum.

7. Utilisation d'une préparation selon l'une quelconque des revendications précédentes, pour le nettoyage et la désinfection d'instruments médicaux dans des "conditions propres" et/ou des conditions "sales" ("*clean*" et/ou "*dirty*").
